# EUROPÄISCHE PATENTSCHRIFT

(11) **EP 2 253 342 B1**
(45) Veröffentlichungstag und Bekanntmachung des Hinweises auf die Patenterteilung: **20.11.2013**
(21) Anmeldenummer: 10009424.2
(22) Anmeldetag: 21.03.2003
(51) Int. Cl.: A61M 1/34, A61M 1/16

(54) **Vorrichtung zur extrakorporalen Blutbehandlung**
Apparatus for extracorporeal blood treatment
Appareil pour traitement extracorporel du sang

(30) Priorität: 27.04.2002 DE 10218846
(43) Veröffentlichungstag der Anmeldung: 24.11.2010
(62) Teilanmeldung aus: 03006422.4
(73) Patentinhaber: Fresenius Medical Care Deutschland GmbH, 61352 Bad Homburg v.d.H. (DE)
(72) Erfinder: Zhang, Wei, Dr.-Ing., 97464 Niederwerrn (DE); Müller, Carsten, 97502 Euerbach (DE)
(74) Vertreter: Oppermann, Frank

(56) Entgegenhaltungen:
- DE-A1- 4 239 937
- US-A1- 2001 045 395

## Beschreibung

Die Erfindung betrifft eine Vorrichtung zur extrakorporalen Blutbehandlung mit einer Einrichtung zur Unterbrechung oder Fortführung der extrakorporalen Blutbehandlung mit veränderten Flussraten.

Zur Entfernung von hampflichtigen Substanzen und zum Flüssigkeitsentsentzug werden beim chronischen Nierenversagen verschiedene Verfahren zur extrakorporalen Blutbehandlung bzw. -reinigung eingesetzt. Bei der Hämodialyse wird das Blut des Patienten ausserhalb des Körpers in einem Dialysator gereinigt. Der Dialysator weist eine Blutkammer und dialysierflüssigkeitskammer auf, die von einer semipermeablen Membran getrennt sind. Während der Behandlung strömt das Blut des Patienten durch die Blutkammer. Um das Blut effektiv von den hampflichtigen Substanzen zu reinigen, wird die Dialysierflüssigkeitskammer kontinuierlich von frischer Dialysierflüssigkeit durchströmt.

Während bei der Hämodialyse (HD) der Transport der kleinmolekularen Substanzen durch die Membran im wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei einer Hämofiltration fungiert der Dialysator als Filter. Eine Kombination aus beiden Verfahren ist die Hämodiafiltration (HDF).

Die US 2001/0045395 A1 beschreibt eine extrakorporale Blutbehandlungsvorriehtung insbesondere zur Hämofiltration, die über eine zentrale Steuer- und Regeleinheit verfügt. Die zentrale Steuer- und Regeleinheit gibt die Flussraten der Pumpen vor, mit denen Flüssigkeit über den Hämofilter dem Patienten zugeführt bzw. entzogen werden kann. Die Flussraten können von der Steuer- und Recheneinheit derart vorgegeben und verändert werden, dass dem Patienten eine vorgegebene Menge an Flüssigkeit entzogen wird.

Die US 2001/0045395 A1 beschreibt den Betrieb der extrakorporalen Blutbehandlungsvorrichtung. Zum Ein- und Ausschalten der Blutbehandlungsvorrichtung werden die Pumpen gestartet bzw. gestoppt.

Bei der Hämo(dia)filtration wird ein Teil des durch die Membran abgezogenen Serums durch eine sterile Substitutionsflüssigkeit ersetzt, die entweder stromauf des Dialysators (Prädilution) oder stromab des Dialysators (Postdilution) dem extrakorporalen Blutkreislauf zugeführt wird.

Es sind Vorrichtungen zur Hämo(dia)filtration bekannt, bei denen die Dialysierflüssigkeit online aus Frischwasser und Konzentraten und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf online aus dem Dialysierflüssigkeitssystem der Maschine über eine Substitutionsflüssigkeitsleitung zugeführt. Bei der Prädilution führt die Substitutionsflüssigkeitsleitung zu einer Anschlussstelle an der arteriellen Blutleitung stromauf des Dialysators, während bei der Postdilution die Substitutionsflüssigkeitsleitung zu einer Anschlussstelle an der venösen Blutleitung stromab des Dialysators führt.

Bei der online Blutbehandlung mit Postdilution wird das Serum im extrakorporalen Blutkreislauf über den Dialysator entzogen, so dass sich das Blut im Dialysator verdickt. Das verdickte Blut wird anschliessend durch die Zugabe der Substitutionsflüssigkeit wieder verdünnt, die stromab des Dialysators in die venöse Blutleitung strömt. Dadurch bildet sich zwischen dem Auslass des Dialysators und der Anschlussstelle der Substitutionsflüssigkeitsleitung an die venöse Blutleitung eine Flüssigkeitssäule aus verdicktem Blut. Diese Flüssigkeitssäule stellt für die Blutbehandlung grundsätzlich kein Risiko dar.

Die Erfinder haben aber erkannt, dass unter bestimmten Betriebsbedingungen der Blutbehandlungsvorrichtung die Gefahr besteht, dass verdicktes Blut mit hoher Geschwindigkeit in die Kanüle der venösen Blutleitung gelangt. Dabei kann es zu einer Gefährdung des Patienten aufgrund einer Hämolyse kommen.

Eine der Betriebsbedingungen der Blutbehandlungsvorrichtung, bei der eine Hämolyse nicht auszuschliessen ist, liegt beispielsweise dann vor, wenn die Blutbehandlung unterbrochen wird. In diesem Zusammenhang ist unter einer Unterbrechung der Blutbehandlung auch ein Aussetzen der Behandlung nur für eine sehr kurze Zeitdauer zu verstehen.

Eine Unterbrechung der Blutbehandlung ist beispielsweise dann erforderlich, wenn das Dialysierflüssigkeitssystem mittels eines konventionellen Druckhaltetests auf Undichtigkeiten überprüft wird. Beim Start des Druckhaltetests wird die Substituatpumpe abrupt gestoppt, während noch Dialysierflüssigkeit durch den Dialysator strömt. Anschliessend wird der Dialysator von dem Dialysierflüssigkeitssystem abgetrennt.

Bei den bekannten Druckhaltetests wird im Dialysierflüssigkeitssystem mittels der Ultrafiltrationspumpe ein Unterdruck erzeugt Für den Fall, dass der Druck in einem bestimmten Zeitraum eine vorgegebene Druckbedingungen verfehlt, wird angenommen, dass das System eine Leckage aufweist, Andernfalls wird von der Dichtigkeit des Systems ausgegangen.

Bei der Messung des Drucks in der venösen Blutleitung haben die Erfinder positive Spitzen mit einer bestimmten Periodendauer im Drucksignal beockbachtet. Es hat sich gezeigt, dass diese Periodendauer genau der Dauer einer Periode des Druckhaltetests entspricht, der zyklisch erfolgt. Die Erfinder haben erkannt, das dieses Phänomen darauf zurückzuführen ist, das beim Druckhaltetest kurzfristig verdicktes Blut in die venöse Blutleitung gelangt- Dieses Phänomen konnte auch durch einen kurzfristigen Abfall des relativen Blutvolumen nachgewiesen werden, da das Blut mit dem sehr viel höheren Hämatokrit (HKT) über den Shunt durch Rezirkulation in den arteriellen Blutschlauch zurückströmt. Es hat sich gezeigt, dass der HKT-Anstieg beim Druckhaltetest von Substituat- und Ultrafiltrationsrate abhängig ist. Eine höhere Substituatrate/Ultrafiltrationsrate begünstigt den HKT-Anstieg beim Druckhaltetest.

Eine Hämolyse ist aber auch dann nicht auszuschliessen, wenn die Blutbehandlung zwar nicht unterbrochen, aber mit geänderten Flussraten fortgeführt wird.

Der Erfindung liegt die Aufgabe zugrunde, eine Vorrichtung zur extrakorporalen

Blutbehandlung anzugeben, bei der eine Gefährdung durch Hämolyse auch bei einer Unterbrechung oder Fortführung der Blutbehandlung mit geänderten Flussraten ausgeschlossen ist.

Die Lösung dieser Aufgabe erfolgt erfindungsgemäss mit den Merkmalen des Patentanspruchs 1. Vorteilhafte Ausführungsformen der Erfindung sind Gegenstand der Unteransprüche.

Die erfindungsgemäße Vorrichtung beruht darauf, dass zur Unterbrechung oder Fortfürung der Blutbehandlung mit geänderten Flussraten die Substituatpumpe nicht plötzlich gestoppt bzw. die Flussrate abrupt geändert wird.

Bei einer ersten Ausführungsform wird die Förderrate der Substituatpumpe und die Ultrafiltrationsrate, die sich um die Nettoentzugsrate unterscheiden, mit der Flüssigkeit insgesamt dem extrakorporalen Kreislauf entzogen werden soll, von vorgegebenen Werten während vorgegebener Zeitintervalle reduziert, bevor der Dialysierflüssigkeitsfluss durch den Dialysator oder Filter (Hämofiltration) unterbrochen wird. Die Reduzierung der Förderrate der Substituatpumpe und der Ultrafiltrationsrate kann dabei kontinuierlich oder diskontinuierlich in einzelnen Schritten erfolgen. Vorzugsweise wird die Förderrate der Substituatpumpe und die Ultrafiltrationsrate im gleichen Zeitintervall reduziert. In der Praxis kann es aber ausreichend sein, wenn die Zeitintervalle, in denen die Förderrate der Substituatpumpe und die Ultrafiltrationsrate verringert werden, nicht exakt gleich sind. Die "langsame" Reduzierung der Förderrate der Substituatpumpe und der Ultrafiltrationsrate führt dazu, dass sich in der venösen Blutleitung eine genau abgegrenzte Flüssigkeitssäule aus verdicktem Blut nicht bilden kann oder zumindest derart "verschmiert" wird, dass sie keine Gefährdung mehr darstellen kann. Zur Unterbrechung der Behandlung wird die Förderrate auf Null reduziert.

Eine mögliche Anwendung der erfindungsgenlässen Vorrichtung ist beispielsweise der Übergang von einer Hämodiafiltrationsbehandlung auf eine Hämodialysebehandlung, Bei der Hämodiafiltation laufen die Substituat-, Dialysierflüssigkeits- und Ultrafiltratpumpe mit vorgegebenen Förderraten. Für den Übergang auf die Hämodialyse wird die Förderrate der Substituatpumpe und der Ultratfiltratpumpe langsam reduziert, wobei die Förderrate der Substituatpumpe auf Null und die Förderrate der Ultrafiltratpumpe soweit verringert wird, dass dem extrakorporalen Kreislauf Flüssigkeit mit einer bestimmten Entzugsrate entzogen wird.

Die Vorrichtung zur extrakorporalen Blutbehandlung gemäss der Erfindung verfügt über eine Steuereinrichtung zur Unterbrechung der Blutbehandlung, mit der die Einstellung der Förderrate der Substituatpumpe und der Ultrafiltrationsrate automatisiert ist.

Die erfindungsgemässe Vorrichtung kann in allen Fällen Anwendung finden, in denen die Blutbehandlung zu unterbrechen oder mit geänderten Flussraten fortzuführen ist, wobei unter einer Unterbrechung der Blutbehandlung auch ein nur kurzeitiges Aussetzen der Behandlung verstanden wird. Eine der Hauptanwendungsfälle sind die bekannten Verfahren zur Überwachung des Dialysierflüssigkeitssystems auf Undichtigkeiten. Diese Verfahren können von den konventionellen Druckhaltetests Gebrauch machen, bei denen im Dialysierlüssigkeitssystem ein Unterdruck erzeugt wird. Es sind aber auch alle anderen Verfahren möglich, bei denen der Dialysator oder Filter vom Dialysierflüssigkeitssystem abgetrennt wird. Beispielsweise kann die Integrität des System mit dem aus der DE 42 39 937 A1 bekannten Verfahren bzw. der Vorrichtung überprüft werden.

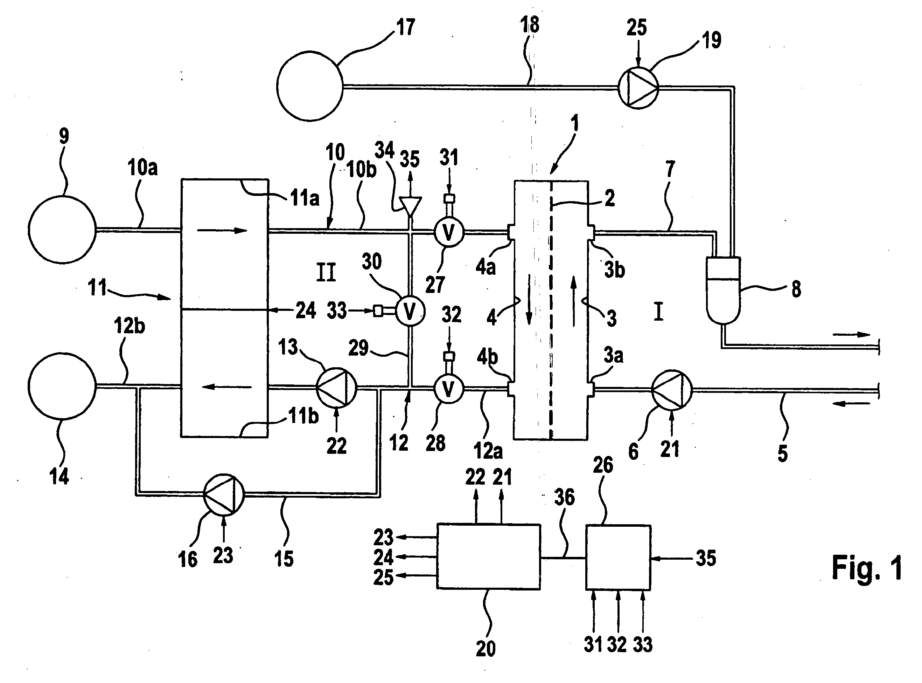

Im folgenden wird ein Ausfuhrungsbeispiel der Erfindung unter Bezugnahme auf die Zeichnung näher erläutert, die in stark vereinfachter schematischer Darstellung die wesentlichen Komponenten einer Hämodialfiltrationsvorrichtung zeugt.

Die Hämodiafiltrationsvorrichtung weist einen Dialysator 1 auf, der durch eine semipermeable Membran 2 in eine Blutkammer 3 und eine Dialysierflüssigkeitskammer 4 getrennt ist. Der Einlass 3a der Blutkammer ist mit einem Ende der arteriellen Blutleitung 5 verbunden, in die eine Blutpumpe 6 geschaltet ist, während der Auslass 3b der Blutkammer mit einem Ende der venösen Blutleitung 7 verbunden ist, in die eine Tropfkammer 8 geschaltet ist. An den anderen Enden der arteriellen und venösen Blutleitung 5,7 befinden sich die nicht dargestellten arteriellen und venösen Kanülen zum Anschluss an den Patienten. Dieser Teil des Flüssigkeitssystems stellt den extrakorporalen Blutkreislauf I der Dialysevorrichtung dar.

Das Dialysierflüssigkeitssystem II der Hämodiafiltrationsvorichtung umfasst eine Einrichtung 9 zur Bereitstellung frischer Dialysierflüssigkeit. Sie ist über einen ersten Abschnitt 10a einer Dialysierflüssigkeitzuführleitung 10 mit dem Einlass der ersten Kammerhälfte 11a einer Bilanziereinrichtung 11 verbunden. Der zweite Abschnitt 10b der Dialysierflüssigkeitzuführleitung 10 verbindet den Auslass der ersten Bilanzierkammerhälfte 11a mit dem Einlass 4a der Dialysierflüssigkeitskammer 4. Der Auslass 4b der Dialysierflüssigkeitskammer 4 ist über den ersten Abschnitt 12a einer Dialysierflüssigkeitsabführleitung 12 mit dem Einlass der zweiten Bilanzierkammerhälfte 11b der Bilanziereinrichtung 11 verbunden. In den ersten Abschnitt 12a der Dialysierflüssigkeitsabführleitung 12 ist eine Dialysierflüssigkeitspumpe 13 geschaltet. Der Auslass der zweiten Bilanzierkammerhälfte 11b ist über den zweiten Abschnitt 12b der Dialysierflüssigkeitsabführleitung 12 mit einem Auslauf 14 verbunden. Stromauf der Dialysierflüssigkeitspumpe 13 zweigt von der Dialysierflüssigkeitsabführleitung 12 eine Ultrafiltratleitung 15 ab, die ebenfalls zu dem Auslauf 14 führt. In die Ultrafiltratleitung 15 ist eine Ultrafiltrationspumpe 16 geschaltet.

In der Figur sind der besseren Übersichtlichkeit halber nur die beiden Kammerhälften der ersten Bilanzkammer dargestellt. Die Bilanziereinrichtung weist aber noch eine zweite Bilanzkammer auf, die der ersten Kammer parallel geschaltet ist. Darüber hinaus weist die Bilanziereinrichtung ebenfalls nicht dargestellte Ventile zur Steuerung des Dialysierflüssigkeitsflusses auf. Derartige Bilanziersysteme sind beispielsweise in der DE 28 38 414 A1 oder DE 197 08 391 C1 beschrieben.

Die Blutkammer 3 wird von dem Blut des Patienten und die Dialysierflüssigkeitskammer 4 des Dialysators 1 von der Dialysierflüssigkeit durchströmt. Da die Bilanziereinrichtung 11 in den Dialysierflüssigkeitsweg geschaltet ist, kann nur soviel Dialysierflüssigkeit über die Dialysierflüssigkeitszuführleitung 10 zufliessen, wie Dialysierflüssigkeit über die Dialysierflüssigkeitsabführleitung 12 abfliessen kann. Mit der Ultrafiltrationspumpe 16 kann dem extrakorporalen Blutkreislauf I über den Dialysator 1 Flüssigkeit entzogen werden.

Darüber hinaus verfügt die Hämodiafiltrationsvorrichtung über eine Substituatquelle 17, von der eine Substituatleitung 18, in die eine Substituatpumpe 19 geschaltet ist, zu der venösen Tropfkammer 8 führt. Mit der Substituatpumpe 19 kann dem extrakorporalen Blutkreislauf I eine vorgegebene Menge an Substitutionsflüssigkeit aus der Substituatquelle 17 zugeführt werden, während dem Blutkreislauf Flüssigkeit über den Dialysator 1 entzogen wird.

Die Hämodiafiltrationsvorrichtung umfasst eine zentrale Steuer- und Regeleinheit 20, die über Steuerleitungen 21 bis 25 mit der Blutpumpe 6, der Dialysierflüssigkeitspumpe 13, der Ultrafiltrationspumpe 16, der Bilanziereinrichtung 11 und der Substituatpumpe 19 verbunden ist. Die Steuer- und Recheneinheit 20 sendet die Steuerbefehle an die einzelnen Komponenten und empfängt von den Komponenten Daten über die Betriebszustände der Komponenten, beispielsweise die Förderraten der Pumpen, die Bilanzierkammertakte, etc.

Zur Überprüfung des Dialysierflüssigkeitsystems II auf Undichtigkeiten weist die Hämodiafiltrationsvorrichtung eine Einrichtung 26 auf, die über eine Datenleitung 27 mit der zentralen Steuer- und Regeleinheit 20 verbunden ist. In die Dialysierflüssigkeitszuführleitung 10 ist stromab der Bilanziereinrichtung 11 ein erstes Absperrventil 27 und in die Dialysierflüssigkeitsabführleitung 12 stromauf der Bilanziereinrichtung 11 ein zweites Absperrventil 28 geschaltet. Stromauf des ersten Absperrventil 27 geht von der Zuführleitung 10 eine Bypassleitung 29 ab, die stromab des zweiten Absperrventils 28 zu der Abführleitung 12 führt. In die Bypassleitung 29 ist ein drittes Absperrventil 30 geschaltet. Die Absperrventile 27, 28, 30 sind elektromagnetisch betätigbare Ventile, die über Steuerleitungen 31, 32, 33 mit der Einrichtung 26 zur Überprüfung des Flüssigkeitssystems verbunden sind. Ferner ist ein Drucksensor 34 vorgesehen, der den Druck in der Dialysierflüssigkeitszuführleitung 10 stromauf des ersten Absperrventils 27 misst. Dieser Drucksensor 34ist über eine Datenleitung 35 ebenfalls mit der Einrichtung 26 verbunden.

Die Überprüfung des Dialysierflüssigkeitssystems II auf Undichtigkeiten erfolgt nach einer Unterbrechung der Dialysebehandlung wie folgt. Die Einrichtung 26 schliesst die Absperrventile 27, 28 und öffnet das Absperrventil 30 für die Dauer des Prüfintervalls Tₚ, so dass die Dialysierflüssigkeitskammer 4 vom Dialysierflüssigkeitssystem II abgetrennt ist. Während des Prüfintervalls Tₚ überwacht die Einrichtung 26 den Betriebsdruck im Dialysierflüssigkeitssystem mit dem Sensor 34 und vergleicht den Betriebsdruck mit einem vorgegebenen Grenzwert. Der Druck steigt während des Prüfintervalls zunächst an, gleicht sich aus und bleibt im Fall eines intakten Systems stabil. Im Fall eines Lecks dagegen fällt er unter den vorgegebenen Grenzwert ab. Dieser Druckabfall ist ein eindeutiger Indikator dafür, dass das UF- Kontrollsystem nicht mehr integer ist. Wenn der Betriebsdruck also den vorgegebenen Grenzwert unterschreitet, erzeugt die Einrichtung 26 ein akkustisches und/oder optisches Alarmsignal. Dieser Druckhaltetest zur Feststellung einer Leckage ist in der DE-A-42 39 937 im Einzelnen beschrieben, auf die ausdrücklich Bezug genommen wird.

Eine Steuereinrichtung 20, die Bestandteil der zentralen Steuer- und Regeleinheit 20 ist, übernimmt vor dem Druckhaltetest die Unterbrechung der Blutbehandlung. Während der Blutbehandlung fördern die Substituatpumpe 19 und die Ultrafiltrationspumpe 16 Dialysierflüssigkeit bzw. Substitutionsflüssigkeit mit einer vorgegebenen Förderrate. Die Steuereinrichtung reduziert über ein vorgegebenes Zeitintervall T langsam die Förderraten beider Pumpen auf Null.

Erst bei Stillstand der Pumpen aktiviert die Steuereinrichtung 20 die Einrichtung 26 zur Überprüfung des Dialysierflüssigkeitssystems II, die den Dialysator vom Dialysierflüssigkeitssystem abtrennt und den Druckhaltetest durchführt.

Da die Substituat- und Ultrafiltrationspumpe 16, 25 nicht abrupt gestoppt werden, kann sich in dem Leitungsabschnitt der venösen Blutleitung 7 zwischen dem Auslass 3b der Blutkammer 3 und der Tropfkammer 8 nicht eine scharf abgegrenzte Flüssigkeitssäule aus verdicktem Blut bilden. Daher besteht auch nicht die Gefährdung des Patienten durch eine Hämolyse.

Die vorgegebene Zeitdauer T, in der die Förderraten reduziert werden, liegt in der Praxis vorzugsweise zwischen 20 und 30 Sekunden. Die Zeitdauer sollte grösser als 10, vorzugsweise 20 Sekunden sein. Sie ist abhängig von der Blutflussrate und dem Füllvolumen des Dialysators sowie des venösen Schlauchleitungsabschnitts. In der Praxis reicht es aber aus, wenn ein bestimmtes Zeitintervall für alle einstellbaren Flussraten vorgegeben ist. Die Zeitdauer kann abhängig von dem Verhältnis Substitutionsflussrate/Blutflussrate berechnet werden, wobei die Abhängigkeit vorzugsweise linear ist.

Bei einer alternativen Ausführungsform arbeitet die Steuereinrichtung wie folgt. Zunächst reduziert die Steuereinrichtung die Ultrafiltrationsrate auf Null, in dem die Ultrafiltrationspumpe 16 abgeschaltet wird. Nach dem Abschalten der Ultrafiltrationspumpe 16 wird die Substituatpumpe 19 noch mit der zuvor eingestellten Förderrate während eines vorgegebenen Zeitintervalls T betrieben, so dass die in der venösen Blutleitung 7 bildende Flüssigkeitssäule aus verdicktem Blut mit der noch zufliessenden Substitutionsflüssigkeit verdünnt wird. Dadurch ist sichergestellt, das verdicktes Blut nicht die venöse Kanüle erreicht. Erst nach dem Stillstand der Substituatpumpe wird die Einrichtung 26 zur Abtrennung des Dialysators und Durchführung des Druckhaltetests aktiviert.

Die vorgegebene Zeitdauer T, in der die Substituatpumpe 19 nach Abschalten der Ultrafiltrationspumpe 16 noch betrieben wird, ist wieder abhängig von dem Füllvolumen des Dialysators 4 und der venösen Blutleitung 7 sowie dem Blutfluss. Die Zeitdauer sollte grösser als 10, vorzugsweise 20 Sekunden sein. In der Praxis liegt das Zeitintervall vorzugsweise zwischen 20 und 30 Sekunden.

## Patentansprüche

1. Vorrichtung zur extrakorporalen Blutbehandlung mit einem extrakorporalen Blutkreislauf (I), der eine erste Kammer (3) eines durch eine semipermeable Membran (2) in die erste Kammer und eine zweite Kammer (4) unterteilten Dialysators (1) oder Filters einschliesst, und einem Flüssigkeitssystem (II), das die zweite Kammer des Dialysators oder Filters einschliesst, wobei dem Blutkreislauf über den Dialysator oder Filter mit einer vorgegebenen Ultrafiltrationsrate Flüssigkeit entzogen und Substitutionsflüssigkeit dem Blutkreislauf stromauf oder Stromab des Dialysators oder Filters mit einer Substituatpumpe (19) zugeführt wird, **dadurch gekennzeichnet, dass** eine Steuereinrichtung (20) zur Unterbrechung der extrakorporalen Blutbehandlung oder Fortführung der extrakörpenden Blutbehandlung mit veränderten Flussraten vorgesehen ist, die derart ausgebildet ist, dass zur Unterbrechung der extrakorporalen Blutbehandlung oder Fortführung der extrakorporalen Blutbehandlung mit veränderten Flussraten die Förderrate der Substituatpumpe (19) und die Ultrafiltrationsrate von vorgegebenen Werten über vorgegebene Zeitintervalle reduziert werden.

2. Vorrichtung nach Anspruch 1, **dadurch gekennzeichnet, dass** die Steuereinrichtung (20) derart ausgebildet ist, dass nach der Reduzierung der Förderrate der Substituatpumpe (19) und der Utrafiltrationsrate der Fluss von Dialysierflüssikeit durch den Dialysator (1) unterbrochen wird.

3. Vorrichtung nach Anspruch 1 oder 2, **dadurch gekennzeichnet, dass** die Steuereinrichtung (20) derart ausgebildet ist, dass die Förderrate der Substituatpumpe (19) und die Ultrafiltrationsrate auf Null reduziert werden.

4. Vorrichtung nach einem der Ansprüche 1 bis 3, **dadurch gekennzeichnet, dass** die vorgegebene Zeitdauer T, in der die Förderrate der Substituatpumpe (19) und die Ultrafiltrationsrate reduziert werden, zwischen 10 und 30 Sekunden, vorzugsweise zwischen 20 und 30 Sekunden liegt.

## Claims

1. Device for extracorporeal blood treatment, having an extracorporeal blood circuit (I) which includes a first chamber (3) of a dialyzer (1) or filter which is subdivided by a semipermeable membrane (2) into the first chamber and a second chamber (4), and having a fluid system (II) which includes the second chamber of the dialyzer or filter, fluid being removed from the blood circuit at a predefined ultrafiltration rate via the dialyzer or filter, and substitution fluid being supplied to the blood circuit upstream or downstream from the dialyzer or filter by means of a substituate pump (19), **characterized in that** a control unit (20) for interrupting the extracorporeal blood treatment or continuing the extracorporeal blood treatment at altered flow rates is provided which is designed in such a way that, for interrupting the extracorporeal blood treatment or continuing the extracorporeal blood treatment at altered flow rates, the delivery rate of the substituate pump (19) and the ultrafiltration rate are reduced from predefined values over predefined time intervals.

2. Device according to Claim 1, **characterized in that** the control unit (20) is designed in such a way that the flow of dialysis fluid through the dialyzer (1) is interrupted after the reduction in the delivery rate of the substituate pump (19) and the ultrafiltration rate.

3. Device according to Claim 1 or 2, **characterized in that** the control unit (20) is designed in such a way that the delivery rate of the substituate pump (19) and the ultrafiltration rate are reduced to zero.

4. Device according to one of Claims 1 through 3, **characterized in that** the predefined time period T during which the delivery rate of the substituate pump (19) and the ultrafiltration rate are reduced is between 10 and 30 seconds, preferably between 20 and 30 seconds.

## Revendications

1. Dispositif pour le traitement extracorporel du sang avec un circuit de circulation sanguine extracorporelle (I) qui contient une première chambre (3) d'un dialyseur (1) ou filtre divisé par une membrane (2) semi-perméable en ladite première chambre et une deuxième chambre (4), et un système de liquide (II) qui contient la deuxième chambre du dialyseur ou filtre, un liquide étant prélevé dans le circuit de circulation sanguine via le dialyseur ou filtre avec un débit d'ultrafiltration prédéfini et un liquide de substitution étant acheminé dans le circuit de circulation sanguine en amont ou en aval du dialyseur ou filtre au moyen d'une pompe à liquide de substitution (19), **caractérisé en ce qu'**il est prévu un dispositif de commande (20) pour interrompre le traitement extracorporel du sang ou pour poursuivre le traitement extracorporel du sang avec des débits d'écoulement modifiées, lequel est conçu de telle sorte que, pour interrompre le traitement extracorporel du sang ou pour poursuivre le traitement extracorporel du sang avec des débits d'écoulement modifiées, le débit de refoulement de la pompe à liquide de substitution (19) et le débit d'ultrafiltration sont diminuées à des valeurs prédéfinies pendant des intervalles de temps prédéfinis.

2. Dispositif selon la revendication 1, **caractérisé en ce que** le dispositif de commande (20) est conçu de telle sorte qu'après la diminution du débit de refoulement de la pompe à liquide de substitution (19) et du débit d'ultrafiltration, l'écoulement du liquide de dialyse à travers le dialyseur (1) est interrompu.

3. Dispositif selon la revendication 1 ou 2, **caractérisé en ce que** le dispositif de commande (20) est conçu de telle sorte que le débit de refoulement de la pompe à liquide de substitution (19) et le débit d'ultrafiltration sont diminuées à zéro.

4. Dispositif selon l'une quelconque des revendications 1 à 3, **caractérisé en ce que** l'intervalle de temps T prédéfini, pendant lequel sont diminuées le débit de refoulement de la pompe à liquide de substitution (19) et le débit d'ultrafiltration, se situe entre 10 et 30 secondes, de préférence entre 20 et 30 secondes.
